# EUROPÄISCHE PATENTANMELDUNG

(11) **EP 0 567 841 A2**
(43) Veröffentlichungstag der Anmeldung: **03.11.1993**
(21) Anmeldenummer: 93106011.5
(22) Anmeldetag: 14.04.1993
(51) Int. Cl.: C07H 19/04, C07H 21/00, C12Q 1/68

(54) **Fluoreszenzmarkierte Verbindungen, ihre Herstellung und Verwendung**

(30) Priorität: 25.04.1992 DE 4213703
(71) Anmelder: MERCK PATENT GmbH, D-64271 Darmstadt (DE)
(72) Erfinder: Pfleiderer, Wolfgang, Prof. Dr., W-7750 Konstanz (DE); Sigmund, Harald, W-7760 Radolfzell 13 (DE); Reckmann, Bernd, Dr., W-6104 Seeheim (DE)

(57) **Zusammenfassung**

Die Erfindung betrifft neue fluoreszenzmarkierte Nucleoside, Nucleotide und Oligonucleotide der allgemeinen Formel I, ein Verfahren zu ihrer Herstellung sowie deren Verwendung zum Nachweis von DNA, RNA und deren Fragmenten: 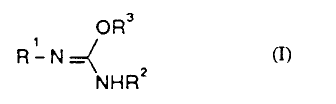
worin
R¹ = 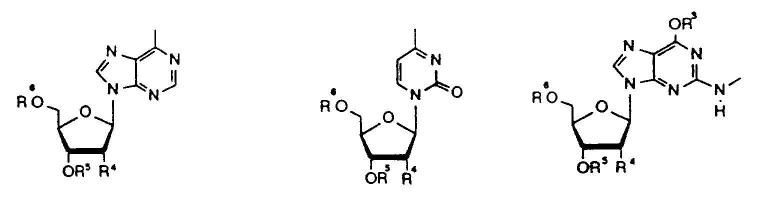
R² = ein Fluorophor
R³ = H, Alkyl mit 1-18 C-Atomen, -CH₂CH₂CN , 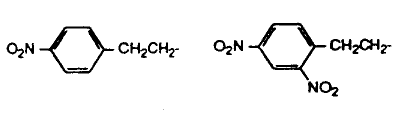
R⁴ = H, OH, OR⁵, OR⁶
R⁵, R⁶ = H, PO(OR⁷)(OR⁸), PS(OR⁷)(OR⁸), R⁸
R⁷ = H, Nucleosid, Nucleotid, Oligonucleotid
R⁸ = OH, übliche Schutzgruppen der Nucleotidchemie bedeuten.

## Beschreibung

Die Erfindung betrifft neue fluoreszenzmarkierte Nucleoside, Nucleotide und Oligonucleotide, ein Verfahren zu ihrer Herstellung sowie deren Verwendung zum Nachweis von DNA, RNA und deren Fragmenten.

Seit der Einführung der ersten Gen-Analysemethode an einem festen Träger durch Southern 1975 spielt die Markierung Von Nucleotiden in der Aufklärung unbekannter DNA bzw. RNA Fragmente eine entscheidende Rolle. Neuere Forschungen auf diesem Gebiet zielen auf einen Ersatz der radioaktiven Nuclide wie ³²P oder ¹²⁵I hin, weil die Abfallproblematik und Sicherheitsvorkehrungen im Umgang mit diesen Substanzen zunehmend kritischer werden. Weitere Nachteile der radioaktiven Markierung sind lange Entwicklungszeiten und kurze Halbwertszeiten des teuren ³²P-Isotops.

Eine Alternative sind Farbstoffmoleküle, die durch Fluoreszenz oder Chemolumineszenz, mit oder ohne Zusatz von Enzymsystemen, eine Bestimmung ermöglichen. Die chemische Sythese von Oligonucleotiden an festen Trägern, die in den letzten Jahren optimiert wurde, bietet zudem eine gute Möglichkeit, die gewünschten Proben-Sequenzen zu erhalten.

Das entscheidende Problem in diesem Zusammenhang ist die Einführung des Markers in die gewünschte Position des Oligomeren. Bislang sind nur solche Methoden bekannt, welche einen Spacer zwischen Nucleosid und Label nutzen, um die Anknüpfung des Markers zu erreichen. Dabei handelt es sich nahezu ausschließlich um modifizierte, schwierig herzustellende Nucleosidbausteine, die durch den Spacer an variierenden Positionen verändert sind und innerhalb eines Synthesezyklus in das Oligomer eingebaut werden Hauptsächlich finden Spacer mit endständigen primären Amino-Funktionen Verwendung, die anschließend mit einem reaktiven Farbstoff umgesetzt werden. Weniger häufig sind Modifikationen mit Thiolfunktionen. Eine weitere Variante ist die Anbindung des Labels an die 3'-bzw. 5'-Position eines Oligonucleotids. Dies bietet dann auch die Möglichkeit von Mehrfachmarkeirungen, wodurch die Nachweisgrenze erheblich herabgesetzt werden kann. (Nucleic Acids Res. 18, 6461 (1990)).

Es ist noch nicht gelungen auf den Spacer zu verzichten; der Effekt einer räumlichen Nähe von Label und Nucleotid auf die Eigenschaften des Farbstoffmoleküls ist bislang unbekannt.

Der Erfindung liegt die Aufgabe zugrunde, fluoreszenzmarkierte Nucleoside, Nucleotide und Oligonucleotide zur Verfügung zu stellen, bei denen der Marker ohne die Verwendung eines Spacers an der gewünschten Position sitzt.

Es wurde überraschenderweise gefunden, daß Aminoschutzgruppen vom Urethan-Typ beim Adenosin und Cytidin in Gegenwart von aliphatischen Aminen in die entsprechenden Harnstoffe übergeführt werden können. Eine analoge Reaktion wurde am N⁶-Phenyloxycarbonyladenosin beobachtet. (J.Chem.Soc.Perkin Trans I, 131 (1978)). Weiterhin ist erstaunlich, daß die Bildung eines Harnstoffs auch mit reaktionsträgen aromatischen Aminen bewerkstelligt werden kann.

Gegenstand der Erfindung sind Verbindungen der allgemeinen Formel I 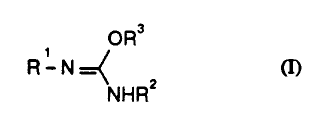
worin
R¹ = 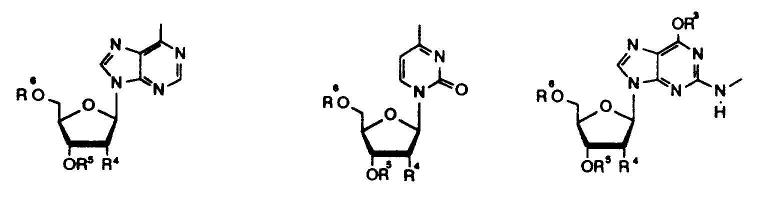
R² = ein Fluorophor
R³= H, Alkyl mit 1-18 C-Atomen, -CH₂CH₂CN , 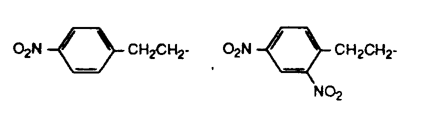
R⁴ = H, OH, OR⁵, OR⁶
R⁵, R⁶ = H, PO(OR⁷)(OR⁸), PS(OR⁷)(OR⁸), R⁸
R⁷ = H, Nucleosid, Nucleotid, Oligonucleotid
R⁸ = OH, übliche Schutzgruppen der Nucleotidchemie
bedeuten.

Die bevorzugten Fluorophore sind solche mit
R² = 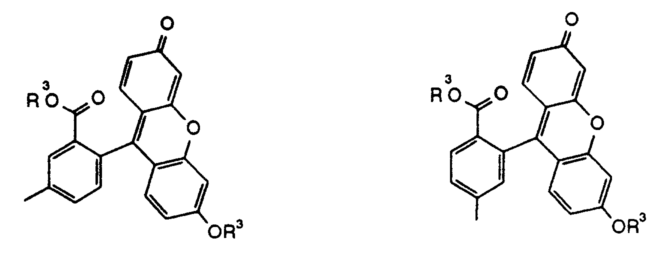
worin
R³ = H, Alkyl mit 1-18 C-Atomen, -CH₂CH₂CN , 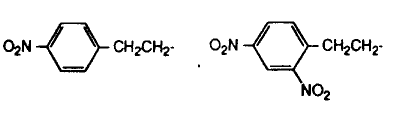
bedeuten. Der Alkylrest in R³ hat vorzugsweise 1-6 C-Atome, besonders bevorzugt sind Methyl- und Äthylgruppen.
Weitere geeignete Fluorophore nach der Erfindung sind Farbstoffe mit
R² = 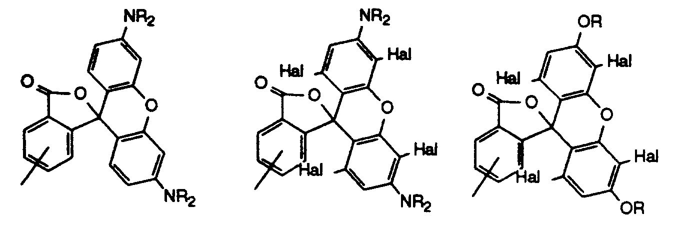
R = Alkyl mit 1-18 C-Atomen, Hal = F, Cl, Br, J
und Acridin-, Pyren-, Psoralen-, Cumarin- Verbindungen mit geeigneter Fluoreszenz.

Bekannte und in der Nucleotidchemie übliche Schutzgruppen (R⁸) sind z.B.
Acetyl-, Benzoyl-, Anisoyl-, Trityl-, Monomethoxytrityl-, Dimethoxytrityl-, p-Nitrophenylethyl-, p-Nitrophenylethoxycarbonyl, 2,4-Dinitrophenylethoxycarbonyl, o-Chlorphenyl- und Methylgruppen.

Ein weiterer Gegenstand der Erfindung ist ein Verfahren zur Herstellung der Verbindungen der allgemeinen Formel I, das dadurch gekennzeichnet ist, daß man Verbindungen der allgemeinen Formel II

R¹-NH₂ (II)

worin R¹ die angegebene Bedeutung hat und R⁷ und R⁸in der Nucleosidchemie übliche Schutzgruppen sind, mit Verbindungen der allgemeinen Formel III

R⁹-CO-R¹⁰ (III)

worin
R⁹ = Cl, Imidazol, Benzotriazol, 1.2.3.4-Tetrazol-, 1.2.3-Triazol-, 1.2.4-Triazolrest, die in 5-Stellung durch Arylreste substituiert sein können und
R¹⁰ = OR¹¹, Imidazol, Benzotriazol mit
R¹¹ = 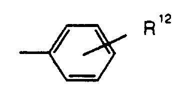
wobei R¹² = O-Alkyl, CN, NO₂, COO-Alkyl, Halogen bedeuten,
mit einem aromatischen Amin der allgemeinen Formel IV,

R²-NH₂ (IV)

worin R² die angegebene Bedeutung hat, umsetzt und die angegebenen Reste R³ einführt und anschließend die Schutzgruppen abgespaltet oder die Zuckerschutzgruppen direkt entfernt.

Die Herstellung der Verbindungen der allgemeinen Formel III erfolgt nach analogen in der Literatur beschriebenen Verfahren (Tetrahedron Letters 1935 (1977)), indem man einen Chlorkohlensäureester der Formel 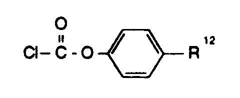
worin R¹² die oben angegebene Bedeutung hat, mit einem gegebenenfalls in 5-Stellung durch einen Arylrest substituiertes Triazol oder Tetrazol in einem inerten Lösungsmittel wie Dioxan oder Methylenchlorid bei 0° bis 25°C umsetzt.

Die Umsetzung von Verbindungen der allgemeinen Formel II mit solchen der allgemeinen Formel III unter Bildung von Urethanen erfolgt in organischen Lösungsmitteln wie Dioxan oder Pyridin bei Temperaturen von 20° bis 40°C. Die weitere Umsetzung der so erhaltenen Urethane mit Verbindungen der allgemeinen Formel IV führt unter analogen Reaktionsbedingungen zu den erfindungsgemäßen Harnstoffen der allgemeinen Formel I.

Der Aufbau von Oligonucleotiden kann sowohl durch Reaktionen in Lösung als auch durch Synthese am festen Träger erzielt werden; letztere Methode wird ab drei monomeren Bausteinen interessant. Von prinzipiell drei Möglichkeiten, der Phosphotriestermethode, der H-Phosphonatmethode und der Phosphitamidmethode (Tetrahedron Letters 31, 2549 (1990)) haben sich die letzteren beiden für Festphasensynthesen durchgesetzt.

Die Oligodeoxyribonucleotidsynthese an Nucleosid-funktionalisiertem Glas nach der Phosphitamidmethode verwendet für den Schutz der vier Nucleosidbasen (Permanentschützung) z.B. basenlabile Schutzgruppen der folgenden Formeln: 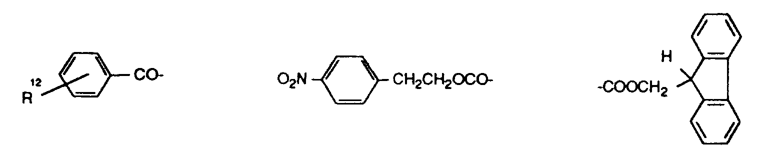
wobei R¹² die oben angegebene Bedeutung hat.

Der intermediäre Schutz der 5'-Position der Ribose wird durch säurelabile Gruppen wie die Monomethoxytrityl- oder die Dimethoxytritylgruppe gewährleistet.

Der entscheidende Punkt bei der Einführung des Markers in ein Oligonucleotid ist die gezielte Freisetzung der zu markierenden Aminofunktion innerhalb eines voll geschützten Oligomeren, d.h. die betreffende Aminofunktion muß während des Oligonucleotidaufbaus zunächst maskiert sein, wobei an die nachfolgende Abspaltungsreaktion dieser Schutzgruppe folgende Bedingungen gestellt werden müssen:
- Säurestabilität bezüglich der Abspaltung der intermediären Schutzgruppen vom Typ der Triphenylmethylgruppen während der Oligonucleotidsynthese.
- Stabilität gegenüber Basen, die als Hilfsbasen bei der Synthese der Phosphitamide benötigt werden.
- Labilität gegenüber Basen, die zur Abspaltung dieser Gruppe eingesetzt werden. Hierbei können keine Basen verwendet werden bei denen der oben angegebene Permanentschutz angegriffen wird. Weiterhin dürfen keine nucleophilen Basen wie Hydroxyde oder primäre/sekundäre Amine eingesetzt werden, da diese die Abspaltung des Oligomeren vom festen Träger verursachen würden.

Eine bevorzugte Lösung dieses Problems bietet die in der Peptidchemie entwickelte und für spezielle Probleme auf die Nucleinsäurechemie übertragene Fluorenylmethoxycarbonyl-Schutzgruppe (FMOC). Für die jeweiligen Abspaltungsreaktionen ergeben sich dann die folgenden Bedingungen:
1. Die Entschützung der 5'-Position während der Festphasensynthese wird mit schwachen Säuren wie Trichloressigsäure bzw. Trifluoressigsäure bewerkstelligt, was gleichzeitig mögliche Depurinierungsreaktionen nahezu ausschließt.
2. Die Abspaltung der FMOC-Schutzgruppe erfolgt mit Triethylamin in Acetonitril oder Pyridin.
3. Die Entschützung der Basen und der Phosphatgruppen wird mit DBU (Diazabicyclo-[5.3.0]-undecan) in Pyridin durchgeführt.
4. Die Abspaltung vom Träger gelingt mit wässrigem Ammoniak.

Die Herstellung des erfindungsgemäß markierten nachstehenden Trimeren in Lösung wird beispielsweise wie folgt durchgeführt: 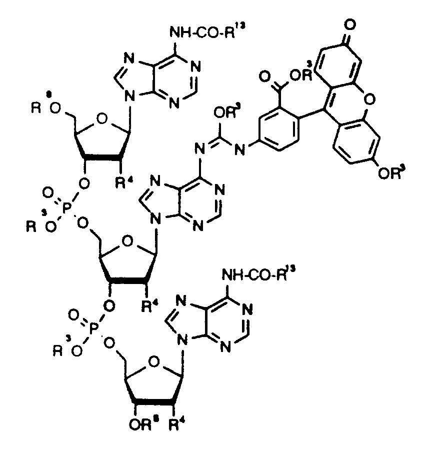
R¹³ = -OCH₂CH₂CN 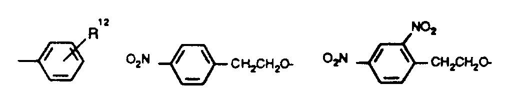
Ein Ausgangsnucleosid der allgemeinen Struktur II mit R⁴,R⁶ = H und den oben erwähnten Nucleosid-Schutzgruppen für den Permanentschutz wird in einem inerten Lösungsmittel wie Methylenchlorid unter Schutzgasbedingungen mit einem Phosphitamid der nachstehenden Struktur 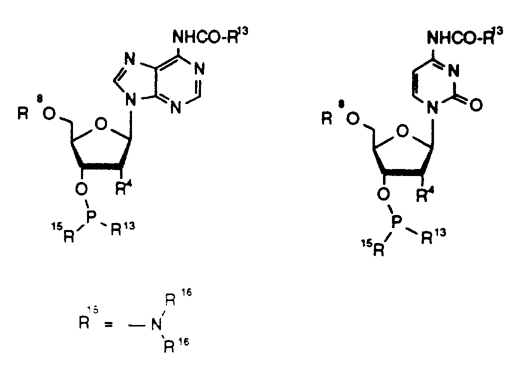
R¹⁶ = Methyl, Ethyl, Propyl, Isopropyl
1-2 Stunden tetrazolkatalysiert bei RT gerührt.

Die Oxidation des dreiwertigen Phosphors erfolgt durch Zugabe von Jod in Pyridin/Wasser und das resultierende dimere Nucleotid der Formel 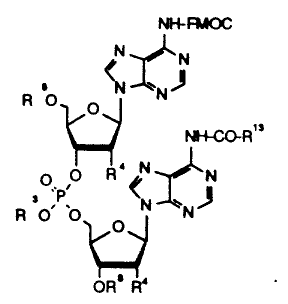
wird durch chromatographische Methoden isoliert. Die 5'-Schutzgruppe wird durch Behandlung mit Toluolsulfonsäure in Methylenchlorid/Methanol abgespalten und so das nachstehende Dimer erhalten. 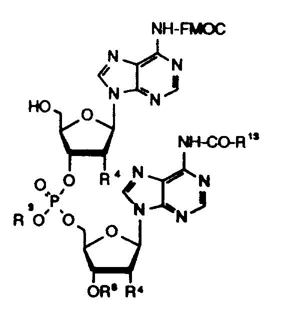
Zur Anknüpfung des dritten Bausteins wird die oben beschriebene Reaktionsfolge mit einem weiteren Phosphitamid der oben aufgefürten Struktur wiederholt. Die Isolierung der resultierenden nachstehenden Verbindung erfolgt erneut chromatographisch. 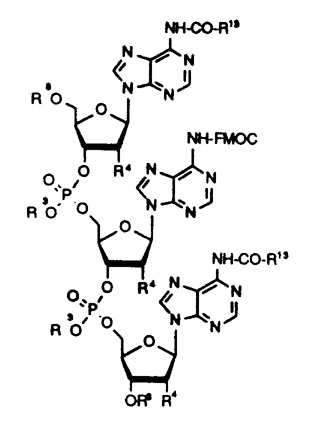
Für die Einführung des Markers sind die folgenden Umsetzungen erforderlich:
Ein Trimeres der oben angegebenen Formel wird mit Triethylamin in Acetonitril entschützt, chromatographisch isoliert und mit einem Tetrazolid der allgemeinen Formel III in Dioxan umgesetzt. Anschließend erfolgt die Reaktion mit einem aromatischen Amin der Formel R²-NH₂ in Pyridin bzw. Dioxan. Nach einem Reinigungsschritt erfolgt die Abspaltung der Schutzgruppen in Pyridin mit DBU und die Isolierung des markierten Trimeren der folgenden Struktur 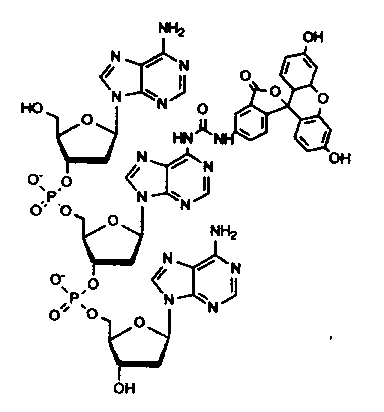
nach Reinigung über Sephadex-FPLC mit Triethylammoniumbikarbonat (TBK) -Puffer und anschließender Überführung des Triethylammoniumsalzes in das Ammoniumsalz durch Papierchromatographie mit Isopropanol/Ammoniak/Wasser oder als Natriumsalz. Das Endprodukt wird zuletzt lyophilisiert bzw. abgesaugt.

Die Herstellung eines erfindungsgemäß markierten Trimeren am festen Träger wird beispielsweise wie folgt durchgeführt:
Zunächst wird ein käuflicher CPG-Träger (controlled pore glass) mit OH- und/oder NH₂-Gruppen mit einem Kondensationsmittel der allgemeinen Formel III und einem Amin der Formel

R¹⁶-NH-(CH₂)ₙ-NH-R¹⁶

worin R¹⁶ = H, Methyl, n = 6 bis 12
bedeuten, umgesetzt, um einen Spacer zwischen Trägermaterial und Nucleosid einzuführen. Die Anknüpfung des ersten Nucleosids an den Spacer erfolgt durch Kondensation mit einem Nucleosid der nachstehenden Formel 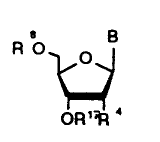
worin B = 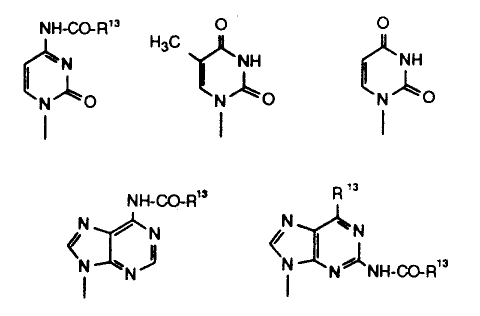
R¹⁷ = -COCH₂CH₂COOH
Die Kondensation erfolgt in Gegenwart eines üblichen Kondensationsmittels, z.B. Dicyclohexylcarbodiimid, in einem Lösungsmittel wie Acetonitril und anschließender Acetylierung reaktiver Restfunktionen wie -OH/-NHR¹⁶ mit Acetanhydrid.

Zum Aufbau des erfindungsgemäß markierten Trimeren wird am DNA-Synthesizer zweimal mit einem Phosphitamid der nachstehenden Formel kondensiert. 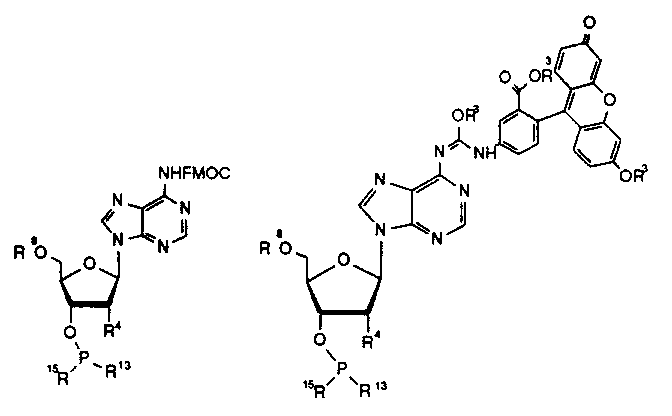
Falls ein FMOC-modifiziertes Phosphitamid mit verwendet wird, erfolgt eine Behandlung des Trägers mit
1. Triethylamin
2. einem Aktivierungsmittel der allgemeinen Formel III
3. einem Amin der allgemeinen Formel IV
Wenn der Marker in das Zielmolekül eingebaut ist, gelingt die Abspaltung aller Schutzgruppen mit aprotischen Basen wie z.B. DBU und anschließend die Ablösung vom Trägermaterial mit Ammoniak.

Mit Hilfe des oben beschriebenen Verfahrens unter Einsatz nachfolgend aufgefühter Phosphitamide ist man in der Lage markierte Oligonucleotide beliebiger Sequenz aufzubauen. 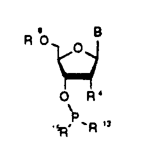
worin B = 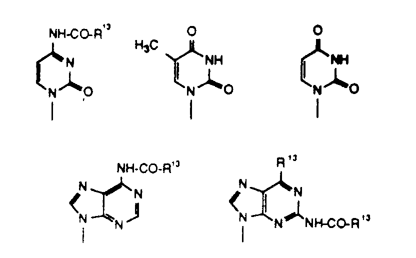 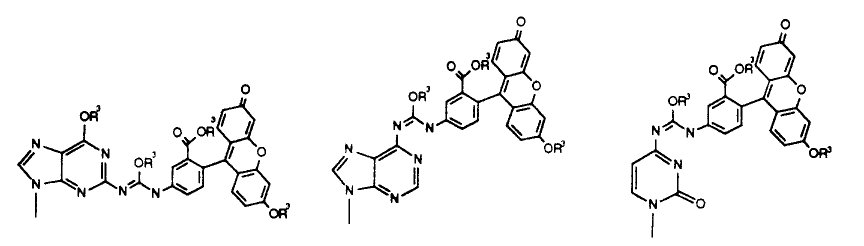
Die erfindungsgemäßen fluoreszenzmarkierten Oligonucleotide unten aufgeführter Struktur eignen sich hervorragend als Sonden zur Untersuchung von Hybridisierungsphänomenen, als Primer zur manuellen und automatischen Sequenzanalyse und zum Nachweis von DNA, RNA und deren Fragmenten. 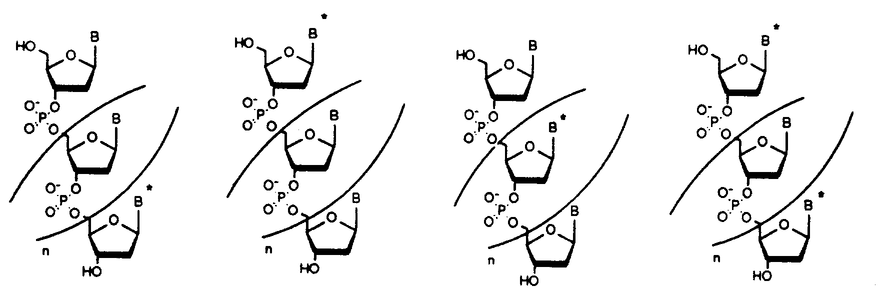
B* = markierte Base
B = Adenin, Guanin, Thymin, Uracil, Cytosin
Zum Nachweis von DNA, RNA und deren Fragmenten wird die zu untersuchende Probe mit dem synthetisch hergestellten, erfindungsgemäß markierten Oligomeren der oben aufgeführten Strukturen und einer Länge von bis zu 50 Basen vermischt.
Der eigentliche Hybridisierungsschritt zwischen der erfindungsgemäß derivatisierten Nucleinsäure und der komplementären Nucleinsaüre in der zu analysierenden Probe erfolgt nach bekannten Methoden. So kann die in der Probe enthaltene Nucleinsäure durch Denaturierung in Einzelstränge getrennt werden und die entstandenen Einzelstränge werden anschließend auf eine Oberfläche wie Nitrozellulose, Nylonmembran oder Mikrotiterplatte fixiert. Die fixierten Einzelstränge werden mit dem komplementären, erfindungsgemäß markierten Oligonucleotid unterhalb der Dissoziationstemperatur in Kontakt gebracht, so daß eine Doppelstrangbildung aus den fixierten Einzelstrangsequenzen und dem komplementären Oligonucleotid ermöglicht wird. Nach den üblichen Waschschritten zum Entfernen des markierten Oligonucleotids wird die Hybridisierung durch Messung der Fluoreszenz des erfindungsgemäßen Labels nachgewiesen.
Alternativ kann das erfindungsgemäß markierte Oligonucleotid in anderen, dem Fachmann bekannten Hybridisierungsmethoden (Anal.Biochem.,1988,169,1-25) eingesetzt werden und durch Messung der Fluoreszenz oder der Fluoreszenzpolarisation nachgewiesen werden.

### Beispiel 1

### N⁴-Phenyloxycarbonyl-2',3',5'-tri-O-acetylcytidin

### Variante A:

3,7 g (10 mmol) 2',3',5'-Tri-O-acetylcytidin und 2,5 g (13 mmol) Phenyloxycarbonyltetrazolid werden in 50 ml Dioxan 30 min bei 40°C gerührt. Die Reaktionsmischung wird eingeengt und mit 30 ml abs. Methylenchlorid aufgefüllt. Nach 1h im Eisfach wird das Tetrazol abgesaugt, das Filtrat eingeengt und auf eine Säule aufgetragen. Die säulenchromatographische Reinigung erfolgt mit 50 g Flash-Kieselgel in Toluol und einer Elutionsfolge von 200 ml Toluol ; 200 ml Tol/Aceton 9:1 ; 200 ml Tol/Aceton 8:2 ; 600 ml Tol/Aceton 7:3.
Die Fraktionen 7-12 werden zu einem farblosen Schaum eingeengt (4,9 g, 100%). Die so erhaltene Verbindung enthält ca 10% Tetrazol, das auch durch eine zweite chromatographische Reinigung nicht entfernt werden kann, für weitere Umsetzungen ist diese Reinheit jedoch ausreichend. Zur Darstellung analysenreiner Substanz eignet sich Methode B.

### Variante B:

740 mg (2 mmol) 2',3',5'-Tri-O-acetylcytidin und 620 mg (2,2 mmol) p-Nitrophenyloxycarbonyltetrazolid werden in 10 ml Dioxan 30 min bei 40°C gerührt. Die Reaktionsmischung wird eingeengt und auf eine Säule aufgetragen. Die säulenchromatographische Reinigung erfolgt mit 20 g Flash-Kieselgel in Toluol und einer Elutionsfolge von 50 ml Toluol ; 200 ml Tol/Aceton 9:1 ; 600 ml Tol/Aceton 8:2.
Die Fraktionen 13-16 werden zu einem farblosen Schaum eingeengt (600 mg, 61 %). Rf= 0,5 (Kieselgel Tol/Aceton 1:1)

### Beispiel 2

### 5-Aminofluoresceinmethylester

3 g 5-Aminofluorescein (8,6 mmol) werden mit 60 ml Methanol und 4 ml konz. Schwefelsäure 40 h unter Rückfluß gekocht. Die dunkelbraune Lösung wird mit 300 ml Eis versetzt und unter Rühren mit ca. 75 ml gesättigter Natriumhydrogencarbonatlösung auf pH 6,5 eingestellt. Es fällt ein orangebrauner Niederschlag aus, der abzentrifugiert wird. Der Niederschlag wird zweimal mit Wasser gewaschen und nach erneutem zentrifugieren mit 100 ml Methanol aufgekocht. Die Suspension wird nach 3h abgesaugt und bei 50°C am Hochvakuum getrocknet. Ausbeute: 2,2 g (6,1 mmol 70%).
Smp.: 284°C Zers.

### Beispiel 3

### N⁴-[Fluoresceinmethylester-5-yl-aminocarbonyl]-2',3',5'-tri-O-acetylcytidin

200 mg 5-Aminofluoresceinmethylester (0,55 mmol) und 500 mg N⁴-Phenyloxycarbonyl-2',3',5'-tri-O-acetylcytidin (1 mmol) werden in 10 ml abs. Pyridin 2h bei 70°C gehalten, anschließend eingeengt, mit Dioxan versetzt und erneut eingeengt. Der Rückstand wird in 20 ml Dioxan in der Wärme im Ultraschallbad behandelt, der Niederschlag abgesaugt und mit Ether gewaschen. Das Rohprodukt wird in ca. 50 ml Dioxan/Methanol 1:1 warm gelöst, filtriert und das Methanol abgezogen. Es resultieren 260 mg (0,34 mmol, 63%) analysenreines Produkt. Smp.: Zersetzung unter Gasentwicklung bei 188-190°C

### Beispiel 4

### N⁶-[Fluorescein-5-yl-aminocarbonyl]-2',3',5'-tri-O-acetyladenosin

347 mg 5-Aminofluorescein (1 mmol) und 1 g N⁶-Phenyloxycarbonyl-2',3',5'-tri-O-acetyladenosin (2 mmol) werden in 10 ml Pyridin 1h bei 70°C gehalten. Die Reaktionslösung wird zur Trochne eingeengt, mit Toluol versetzt und erneut eingeengt. Die säulenchromatographische Reinigung erfolgt mit 50 g SiO₂ (Flash) in Toluol und einer Elutionsfolge von 200 ml Tol/EE 8:2 ; 200 ml Tol/EE 1:1 ; 200ml Tol/EE 1:1 + 10 ml MeOH ; 200 ml Tol/EE 1:1 + 15 ml MeOH ; 200 ml Tol/EE 1:1 + 20 ml MeOH ; 200 ml Tol/EE/MeOH 1:1:1. Die Fraktionen 9 bis 20 werden am Rotationsverdampfer eingeengt, wobei das Produkt aus Toluol ausfällt. Nach Trocknen am Hochvakuum bei RT ergeben sich 780 mg (100%) Produkt. Zur Darstellung analysenreiner Substanz wird das erhaltene Produkt in 5 ml Isopropanol erhitzt und über Nacht stehen gelassen. Der Niederschlag wird vom Kolbenrand gelöst, abgesaugt und getrocknet (400mg). Die Mutterlauge wird mit ca. 300 ml Ether versetzt und bei -15°C über Nacht gelagert. Der voluminöse Niederschlag wird abgesaugt und mit dem obigen vereinigt. Ausbeute an analysenreiner Substanz: 610 mg (79%).
Smp.: Zersetzung unter Gasentwicklung 188-190°C

### Beispiel 5

### 2'-Desoxy-5'-O-dimethoxytrityl-N⁶-fluorenylmethoxycarbonladenosin-3'-O-phosphorigsäure-2-(p-nitrophenyl)ethylester-N,N-diisopropylamid

Unter Schutzgasbedingungen werden 3 g 5'-O-Dimethoxytrityl-N⁶-fluorenylmethoxycarbonyl-2'-desoxyadenosin (3,9 mmol) in 20 ml Methylenchlorid und 3 ml (17 mmol) Diisopropylethylamin gelöst. Im N₂-Gegenstrom werden 3 g Phosphorigsäurechlorid-2-(p-nitrophenyl)ethylester-N,N-diisopropylamid (9 mmol) zugesetzt und die gelbe Mischung 70 min bei Raumtemperatur (RT) unter Stickstoff gerührt. Die orange Reaktionsmischung wird mit 400 ml Chloroform verdünnt und die org. Phase mit 70 ml Phosphatpuffer pH7 ausgeschüttelt. Die Wasserphase wird noch einmal mit Chloroform gewaschen und anschließend wird die org. Phase über Magnesiumsulfat getrocknet, zu einem braunen Öl eingeengt und am Hochvakuum die restliche Hünig-Base entfernt. Das Rohprodukt wird in wenig Methylenchlorid gelöst und auf eine Alox-Säule aufgegeben.
Die chromatographische Reinigung erfolgt mit 150 g Alox (neutral) in Petrolether und einer Elutionsfolge von 100 ml Petrolether ; 380 ml Petrolether + 20 ml Aceton ; 360 ml Petrolether + 40 ml Aceton ; 340 ml Petrolether + 60 ml Aceton ; 320 ml Petrolether + 80 ml Aceton; 300 ml Petrolether + 100 ml Aceton ; 100 ml Petrolether + 100 ml Aceton. Die Fraktionen 26-40 enthalten das Produkt, welches aus abs. Methylenchlorid aufgeschäumt wird. Ausbeute: 2,9 g (2,7 mmol, 70%)
Rf=0,45 (Alox, neutral, Petrolether/Aceton 8/2)

### Beispiel 6

### 2'-Desoxy-5'-O-dimethoxytrityl-N⁶-fluorenylmethoxycarbonyladenylyl-[3'-{O^{P}-2-(p-nitriophenyl)ethyl}-5']-N⁶,3'-O-bis-[2-(p-nitrophenyl)ethoxycarbonyl]-2'-desoxyadenosin

2,9 g 2'-Desoxy-5'-O-dimethoxytrityl-N⁶-fluorenylmethoxycarbonyladenosin-3'-O-phosphorigsäure-2-(p-nitrophenyl)ethylester-N,N-diisopropylamid (2,7 mmol) und 1,4 g N⁶,3'-O-Bis-[2-(p-nitrophenyl)ethoxycarbonyl]-2'-desoxyadenosin (2,2 mmol) werden eingewogen und unter Sauerstoffausschluß in 10 ml abs. Acetonitril und 5 ml abs. Methylenchlorid gelöst. Im N₂-Gegenstrom werden 920 mg Tetrazol (15 mmol) zugesetzt und die Mischung 1,5h bei RT gerührt. Anschließend wird mit 1,2g Jod in Pyridin/Methylenchlorid/Wasser 15 min. oxidiert. Die dunkelbraune Reaktionsmischung wird mit 400 ml Chloroform verdünnt und durch Extraktion mit Natriumthiosulfat/Natriumchloridlösung entfärbt. Die org. Phase wird über Magnesiumsulfat getrocknet, zu einem gelben Öl eingeengt und mit Toluol versetzt und erneut eingeengt bis der Pyridingeruch verschwunden ist. Das Rohprodukt wird in wenig Methylenchlorid gelöst und auf eine Kieselgel-Säule aufgegeben. Die chromatographische Reinigung erfolgt mit 60 g Flash-SiO₂ in Toluol und einer Elutionsfolge von 100 ml Toluol ; 200 ml Toluol/Aceton 9:1 ; 200 ml Toluol/Aceton 8:2 ; 200 ml Toluol/Aceton 7:3 ; 400 ml Toluol/Aceton 6:4 ; 200 ml Toluol/Aceton 1:1 ; 200 ml Toluol/Aceton 4:6 ; 100 ml Methanol. Fraktionen 27-43 enthalten das Produkt, welches aus abs. Methylenchlorid aufgeschäumt wird. Ausbeute: 2,8g (1,72 mmol, 78%)
Zur Analyse muß diese Substanz zweimal mit Ethanol und ca. dreimal mit Methylenchlorid versetzt und erneut eingeengt, um das Toluol zu entfernen.
Rf=0,61 (Kieselgel Tol/EE/MeOH 5:4:1) ; Rf=0,31 (Kieselgel Tol/Aceton 1:1)

### Beispiel 7

### 2'-Desoxy-N⁶-fluorenylmethoxycarbonyladenylyl-[3'-{O^{P}-2-(p-nitrophenyl)ethyl}-5']-N⁶,3'-O-bis-[2-(p-nitrophenyl)ethoxycarbonyl]-2'-desoxyadenosin

1,0 g 2'-Desoxy-5'-O-dimethoxytrityl-N⁶-fluorenylmethoxycarbonyladenylyl-[3'-{O^{P}-2-(p-nitrophenyl)ethyl}-5']-N⁶,3'-O-bis-[2-(p-nitrophenyl)ethoxycarbonyl]-2'-desoxyadenosin (0,62 mmol) werden mit 30 ml 1,5% Trichloressigsäure in Methylenchlorid 1 h bei RT gerührt. Die Reaktionsmischung wird mit 300 ml Chloroform verdünnt und mit Phosphatpuffer pH7 neutral gewaschen. Die organische Phase wird über Magnesiumsulfat getrocknet und eingeengt. Das Rohprodukt wird in wenig Methylenchlorid auf eine Kieselgelsäule aufgegeben. Die chromatographische Reinigung erfolgt über 30 g Flash-SiO₂ in Toluol und einer Elutionsfolge von 100 ml Toluol ; 200 ml Toluol/EE 1:1 ; 95 ml Toluol/EE 1:1 + 5 ml MeOH ; 90 ml Toluol/EE 1:1 + 10 ml MeOH ; 80 ml Toluol/EE 1:1 +20 ml MeOH ; 80 ml Toluol/EE 1:1 +20 ml MeOH; 80 ml Toluol/EE 1:1 +20 ml MeOH. Die Fraktionen 7 und 8 enthalten das Produkt, welches aus abs. Methylenchlorid aufgeschäumt und am Hochvakuum bei 40°C getrocknet wird. Ausbeute: 780 mg (0,59 mmol, 96%).
Rf=0,2 (Kieselgel Tol/EE/MeOH 5:4:1)

### Beispiel 8

### N⁶,5'-O-Bis-[2-(p-nitrophenyl)ethoxycarbonyl]-2'-desoxyadenylyl-[3'-{O^{P}-2-(p-nitrophenyl)ethyl}-5']-2'-desoxy-N⁶-fluorenylmethoxycarbonyladenlyl-[3'-{O^{P}-2-(p-nitrophenyl)ethyl}-5']-N⁶,3'-O-bis-[2-(p-nitrophenyl)ethoxycarbonyl]-2'-desoxyadenosin

600 mg 2'-Desoxy-N⁶-fluorenylmethoxycarbonladenylyl-[3'-{O^{P}-2-(p-nitrophenyl)ethyl}-5']-2'-desoxy-N⁶,3'-O-bis-[2-(p-nitrophenyl)ethoxycarbonyl]-2'-desoxyadenosin (0,45 mmol) und 847 mg N⁶,5'-O-Bis-[2-(p-nitrophenyl)ethoxycarbonyl]-2'-desoxyadenosin-3'-O-phosphorigsäure-2-(p-nitrophenyl)ethylester-N,N-diisopropylamid (9,1 mmol) werden eingewogen und unter Sauerstoffausschluß in 10 ml abs. Acetonitril und 10 ml abs. Methylenchlorid gelöst. Im N₂-Gegenstrom werden 190 mg Tetrazol (2,7 mmol) zugesetzt und die Mischung 1,5 h bei RT gerührt. Anschließend wird mit 500 mg Jod in Pyridin/ Methylenchlorid/Wasser 15 min. oxidiert. Die braune Reaktionsmischung wird mit 400 ml Chloroform verdünnt und durch Extraktion mit Natriumthiosulfat/Natriumchloridlösung entfärbt. Die org. Phase wird über Magnesiumsulfat getrocknet, zu einem Öl eingeengt, mit Toluol versetzt und erneut eingeengt bis der Pyridingeruch verschwunden ist, wobei ein farbloser Feststoff resultiert. Dieser wird in wenig Methylenchlorid gelöst und auf eine Kieselgelsäule aufgegeben. Die chromatographische Reinigung erfolgt mit 30 g Flash-SiO₂ in Toluol und einer Elutionsfolge von 400 ml Toluol/EE 1:1 ; 195 ml Toluol/EE 1:1 + 5 ml MeOH ; 190 ml Toluol/EE 1:1 + 10 ml MeOH ; 180 ml Toluol/EE 1:1 + 20 ml MeOH ; 170 ml Toluol/EE 1:1 +30 ml MeOH ; 170 ml Toloul/EE 1:1 + 30 ml MeOH ; 160 ml Toluol/EE 1:1 + 40 ml MeOH. Die Fraktionen 10-15 werden zu einem farblosen Feststoff eingeengt, in Essigsäureethylester aufgeschlämmt, abgesaugt und bei 40°C am Hochvakuum getrocknet. Ausbeute: 920 mg (0,42 mmol, 93%)
Rf=0,11 (Kieselgel Tol/EE/MeOH 5:4:1)

### Beispiel 9

### N⁶,5'-O-Bis-[2-(p-nitrophenyl)ethoxycarbonyl]-2'-desoxyadenylyl-[3'{O^{P}-2-(p-nitrophenyl)ethyl}-5']-2'-desoxyadenylyl-[3'-{O^{P}-2-(p-nitrophenyl)ethyl}-5']-N⁶,3'-O-Bis-[2-(p-nitrophenyl)ethoxycarbonyl]-2'-desoxyadenosin

600 mg N⁶,5'-O-Bis-2[-(p-nitrophenyl)ethoxycarbonyl]-2'-desoxyadenylyl-[3'-{O^{P}-2-(p-nitrophenyl)ethyl}-5']-2'-desoxy-N⁶-fluorenylmethoxycarbonyladenylyl-[3'-{O^{P}-2-(p-nitrophenyl)ethyl}-5']-N⁶,3'-O-bis-[2-(p-nitrophenyl)ethoxycarbonyl]-2'-desoxyadenosin (0,28 mmol) werden mit 40 ml abs. Acetonitril/Methylenchlorid 1:1 und 10 ml Triethylamin 4 h bei RT gerührt. Die Reaktionsmischung wird eingeengt und das braune Öl in Methylenchlorid gelöst auf eine Kieselgelsäule aufgetragen. Die chromatographische Reinigung erfolgt mit 30 g Flash-SiO₂ in Methylenchlorid und einer Elutionsfolge von 50 ml Methylenchlorid ; 200 ml Methylenchlorid/Methanol 98:25 ; 200 ml Methylenchlorid/Methanol 95:5 ; 400 ml Methylenchlorid/ Methanol 96:4 ; 200 ml Methylenchlorid/Methanol 9:1. Die Fraktionen 10-19 enthalten das Produkt und eine braune Verunreinigung. Nach Entfernen des Lösungsmittels resultiert ein brauner Rückstand der in 20-30 ml Methylenchlorid gelöst und abfiltriert wird. Zum Filtrat wird bis zur Trübung Ethanol zugesetzt und das Methylenchlorid am Rotationsverdampfer ohne Erwärmen abgezogen. Dabei scheidet sich das gewünschte Produkt am Kolbenrand ab, die braune Ethanollösung wird abdekantiert und verworfen. Der farblose Rückstand wird in Ether aufgeschlämmt und abgesaugt. Ausbeute: 510 mg (0,26 mmol, 94%). Rf=0,55 (Kieselgel Methylenchlorid/MeOH 9:1)

### Beispiel 10

### N⁶,5'-O-Bis-[2-(p-nitrophenyl)ethoxycarbonyl]-2'-desoxyadenylyl-[3'-{O^{P}-2-(p-nitrophenyl)ethyl}-5']-2'-desoxy-N⁶-phenyloxycarbonladenylyl-[3'-{O^{P}-2-(p-nitrophenyl)ethyl}-5']-N⁶,3'-O-bis-[2-(p-nitrophenyl)ethoxycarbonyl]-2'-desoxyadenosin

300 mg N⁶,5'-O-Bis-[2-(p-nitrophenyl)ethoxycarbonyl]-2'-desoxyadenylyl-[3'-{O^{P}-2-(p-nitrophenyl)ethyl}-5']-2'-desoxyadenylyl-[3'-{O^{P}-2-(p-nitrophenyl)ethyl}-5']-N⁶,3'-O-bis-[2-(p-nitrophenyl)ethoxycarbonyl]-2'-desoxyadenosin (0,15 mmol) werden in 5 ml abs. Dioxan und 200 mg Phenyloxycarbonyl-(p-nitrophenyl)tetrazolid (0,6 mmol) 48h bei 40°C gerührt. Die Reaktionsmischung wird eingeengt und in wenig Methylenchlorid gelöst auf eine Säule aufgetragen. Die Chromatographie wird mit 7 g Flash-SiO₂ in Methylenchlorid und einer Elutionsfolge von 50 ml Methylenchlorid ; 100 ml Methylenchlorid/Methanol 97:3 ; 200 ml Methylenchlorid/Methanol 95:5 durchgeführt. Die Fraktionen 7-10 enthalten das Produkt. Beim Einengen wird Essigsäureethylester zugegeben, um das Produkt nach Abziehen des Methylenchlorids am Kolbenrand niederzuschlagen. Die leicht gelbe Ethylacetatlösung wird abdekantiert und verworfen, der farblose Rückstand in Ether aufgeschlämmt und abgesaugt (220 mg analysenrein). Die analoge Aufarbeitung der Mutterlaugen ergibt noch weitere 70 mg leicht mit Reagenzresten verunreinigtes Produkt. Ausbeute: 290 mg (0,14 mmol, 93%)
Rf=0,68/0,63 (Diastereomere, Kieselgel Methylenchlorid/MeOH 9:1)

### Beispiel 11

### N⁶,5'-O-Bis-[2-(p-nitrophenyl)ethoxycarbonyl]-2'-desoxyadenylyl-[3'-(O^{P}-2-(p-nitrophenyl)ethyl}-5']-2'-desoxy-N⁶-[fluorescein-5-yl-aminocarbonyl]adenylyl-[3'-{O^{P}-2-(p-nitrophenyl)ethyl}-5']-N⁶-3'-O-bis-[2-(p-nitrophenyl)ethoxycarbonyl]-2'-desoxyadenosin

150 mg N⁶,5'-O-Bis-[2-(p-nitrophenyl)ethoxycarbonyl]-2'-desoxyadenylyl-[3'-{O^{P}-2-(p-nitrophenyl)ethy}-5']-2'-desoxy-N⁶-phenloxycarbonladeylyl-[3'-{O^{P}-2-(p-nitrophenyl)ethyl}-5']-N⁶,3'-O-bis-[2-(p-nitrophenyl)ethoxycarbonyl]-2'-desoxyadenosin (73 µmol) und 51 mg 5-Aminofluorescein (146 µmol) in 2 ml abs. Pyridin werden 2 h bei 60-70°C gerührt. Die Reaktionsmischung wird eingeengt, mit Toluol versetzt und erneut eingeengt und auf 5 g Kieselgel aufgezogen. Die Chromatographie erfolgt mit 15 g Flash-SiO₂ in Methylenchlorid und einer Elutionsfolge von 50 ml Methylenchlorid/Methanol ; 100 ml Methylenchlorid/Methanol 98:2 ; 100 ml Methylenchlorid/Methanol 95:5 ; 100 ml Methylenchlorid/Methanol 9:1 ; 100 ml Methylenchlorid/ Methanol 1:1. Die Fraktionen 11-13 enthalten das Produkt, das aus Methanol abgesaugt wird. Ausbeute: 75 mg (32 µmol, 44%). Zur Analyse wird über eine präparative Kieselgelplatte im System Methylenchlorid/Methanol 8:2 gereinigt. Gesamtausbeute: 38 mg (16 µmol, 22%).
Rf=0,39 (Kieselgel Methylenchlorid/MeOH 9:1)

### Beispiel 12

### 2'-Desoxyadenylyl-(3',5')-N⁶-[fluorescien-5-yl-aminocarbonyl]-2'-desoxyadenylyl-(3',5')-2'-desoxyadenosin

90 mg N⁶,5'-O-Bis-[2-(p-nitrophenyl)ethoxycarbonyl]-2'-desoxyadenylyl-[3'-{O^{P}-2-(p-nitrophenyl)ethyl}-5']-2'-desoxy-N⁶-[fluorescein-5-yl-aminocarbonyl]adenylyl-[3'-(O^{P}-2-(p-nitropheny)ethyl}-5']-N⁶,3'-O-bis[2-(p-nitrophenyl)ethoxycarbonyl]-2'-desoxyadenosin (3,89x10⁻⁵ mol) werden mit 10 ml 0,5M DBU in Pyridin 3h bei RT gerührt. Die Reaktionslösung wird mit NaH₂PO₄-Lösung versetzt und mit Methylenchlorid extrahiert. Die wässrige Phase wird mit 5 ml konz Ammoniak versetzt und die stark fluoreszierende Lösung nochmals extrahiert, dann abgetrennt und eingeengt. Das Rohprodukt wird über FPLC / Sephadex DAE25 / Tetrabutylammoniumbicarbonat (TBK)-Puffer gereinigt. Die produkthaltigen Fraktionen werden eingeengt und mehrfach mit Wasser versetzt und erneut eingeengt. Der orangefarbene klebrige Rückstand wird in wenig MeOH gelöst und mit 200 mg Natriumiodid in 50 ml Aceton versetzt. Es bildet sich ein orangefarbener Niederschlag, der nach ca. 20 h unter Schutzgas (Wasserausschluß) abgesaugt und am Hochvakuum getrocknet wird. Ausbeute: 21mg (60%)

### Beispiel 13

### 2'-Desoxy-N⁶-phenyloxycarbonyl-3',5'-O-tetraisopropyldisiloxan-1,3-diyl-adenosin

5 g 2'-Desoxy-3',5-tetraisopropyldisiloxan- 1,3-diyl-adenosin (10 mmol) und 3,8 g Phenyloxycarbonyltetrazolid (20 mmol) werden in 30 ml abs. Dioxan 12h bei 40°C gerührt. Die leicht trübe Reaktionslösung wird eingeengt und mit ca. 50 ml Toluol aufgefüllt und der Niederschlag abgesaugt. Das Filtrat wird bis auf 20 ml eingeengt und auf eine Säule aufgetragen. Die säulenchromatographische Reinigung erfolgt mit 70 g SiO₂ (Flash) in Toluol und einer Elutionsfolge von 250 ml Toluol/Essigester 5:1 und 600 ml Toluol/Essigester 2:1. Die Fraktionen 4-9 werden zur Trockne eingeengt und am Hochvakuum getrocknet. Ausbeute: 4,5g (77% bzgl. 2'-Desoxyadenosin). Zur Analyse werden 500 mg in 20 ml Ether warm gelöst, es kristallisieren bei -20°C 300 mg analysenreines Produkt aus.
Smp. 106-107°C

### Beispiel 14

### 2'-Desoxy-N⁶-(fluorescien-5-yl-aminocarbonyl)-3',5'-tetraisopropyldisiloxan-1,3-diyl-adenosin

1,4 g 5-Aminofluorescein (4 mmol) und 2 g 2'-Desoxy-N⁶-phenyloxycarbonyl-3',5'-tetraisopropyldisiloxan-1,3-diyl-adenosin (4 mmol) werden zunächst getrennt mit abs. Pyridin koevaporiert und dann in 10 ml Pyridin 1 h bei 60°C gehalten. Die Reaktionslösung wird zur Trockne eingeengt, mit Toluol verserst und erneut eingeengt und in Methylenchlorid/Methanol gelöst auf 10 g Kieselgel aufgezogen. Die säulenchromatographische Reinigung erfolgt mit 60 g SiO₂ (Flash) in Methylenchlorid und einer Elutionsfolge von 100 ml Methylenchlorid ; 200 ml Methylenchlorid/MeOH 98/2 ; 200 ml Methylenchlorid/MeOH 97/3 ; 200 ml Methylenchlorid/MeOH 96/4 ; 200 ml Methylenchlorid/MeOH 95/5 ; 200 ml Methylenchlorid/MeOH 9/1 ; 100 ml Methylenchlorid/MeOH 8/2. Die Fraktionen 9-11 werden zur Trockne eingeengt, mit Dioxan versetzt und erneut eingeengt und am Hochvakuum getrocknet. Ausbeute: 2,7g 80%
Rf= 0.44 (Kieselgel Tol/EE/MeOH 5:4:1)

### Beispiel 15

### N-{3'-O-[2-(p-Nitrophenyl)ethyl]-fluorescein-[2-(p-nitrophenyl)ethyl]ester-5-yl}-N'-{(2'-desoxy-3',5'-tetraisopropyldisiloxan-1,3-diyl-β-D-ribofuranosyl)purin-6-yl)-O-[2-(p-nitrophenyl)ethyl]-harnstoff

2,7 g 2'-Desoxy-N⁶-(fluorescein-5-yl-aminocarbonyl)-3',5'-tetraisopropyldisiloxan-1,3-diyl-adenosin (3,1 mmol), 4,8 g Triphenylphosphin (18 mmol) und 4,1 g 2-(p-Nitrophenyl)ethanol (24 mmol) werden in 50 ml abs. Dioxan bei 60°C gerührt bis eine klare Lösung entstanden ist. Der tief roten Lösung werden 3 ml (18 mmol) Azodicarbonsäureethylester zugesetzt und noch 1,5 h bei 60°C gehalten. Die Farbe schlägt dabei nach orange um. Zur Aufarbeitung wird das Reaktionsgemisch mit 300 ml Methylenchlorid verdünnt und mit 100 ml Phosphatpuffer pH7 extrahiert. Die organische Phase wird abgetrennt, über Magnesiumsulfat getrocknet und zu einem tief orangefarbenen Öl eingeengt. Das Rohprodukt wird in 20 ml Toluol gelöst und auf eine Kieselgelsäule aufgetragen. Die chromatographische Reinigung erfolgt mit 100 g Flash-SiO₂ in Toluol und einer Elutionsfolge von 200 ml Toluol ; 500 ml Toluol/Essigester 8/2 ; 500 ml Toluol/Essigester 1/1 ; 480 ml Toluol/Essigester 1/1 + 20 ml MeOH ; 450 ml Toluol/Essigester 1/1 + 50 ml MeOH. Die Fraktionen 13-20 werden eingeengt, mit Ethanol versetzt und erneut eingeengt und mit Methanol in Ultraschallbad behandelt. Der resultierende Niederschlag wird noch ca. 1 h in Methanol gerührt, danach abgesaugt und am Hochvakuum getrocknet. Ausbeute: 3,7g (2,8 mmol, 90%)
Smp.: Zersetzung ab 112°C
Rf= 0.69 (Kieselgel Tol/EE/MeOH 5:4:1)

### Beispiel 16

### N-{3'-O-[2-(p-Nitrophenyl)ethyl]-fluorescein-[2-(p-nitrophenyl)ethyl]ester-5-yl}-N'-{(2'-desoxy-β-D-ribofuranosyl)purin-6-yl}-O-[2-(p-nitrophenyl)ethyl]harnstoff

1 g N-{3'-O-[2-(p-Nitrophenyl)ethyl]-fluorescein-[2-(p-nitrophenyl)ethyl]ester-5-yl}-N'-{(2'-desoxy-3',5'-tetraisopropyldisiloxan-1,3-diyl-β-D-ribofuranosyl)purin-6-yl}-O-[2-(p-nitrophenyl)ethyl]-harnstoff (0,76 mmol) werden in 10 ml THF gelöst und mit 400 µl Essigsäure versetzt. Nach Zugabe von 718 mg (2,28 mmol) TBAF (Trihydrat) wird die Mischung 12 h bei RT gerührt. Zur Aufarbeitung wird das Reaktionsgemisch mit 300 ml Methylenchlorid verdünnt und mit 100 ml verd. NaHCO₃-Lösung extrahiert. Die organische Phase wird abgetrennt, über Magnesiumsulfat getrocknet und Zu einem tief orangefarbenen Öl eingeengt. Das Rohprodukt wird in 20 ml Methylenchlorid gelöst und auf eine Kieselgelsäule aufgetragen. Die chromatographische Reinigung erfolgt mit 20 g Flash-SiO₂ in Methylenchlorid und einer Elutionsfolge von 100 ml Methylenchlorid ; 100 ml Methylenchlorid/Methanol 98:2 ; 200 ml Methylenchlorid/Methanol 95:5 ; 200 ml Methylenchlorid/Methanol 9:1. Die Fraktionen 7-10 werden eingeengt, mit Ethanol versetzt und erneut eingeengt und mit Ethanol im Ultraschallbad behandelt. Der resultierende Niederschlag wird noch ca. 1 h in Ethanl gerührt, danach abgesaugt und am Hochvakkum getocknet. Ausbeute: 680 mg (0,63 mmol, 84%).
Smp.: Zersetzen ab 133°C
Rf= 0,35 (Kieselgel Methylenchlorid/MeOH 9:1)

### Beispiel 17

### N-{3'-O-[2-(p-Nitrophenyl)ethyl]-fluorescien-[2-(p-nitrophenyl)ehtyl]ester-5-yl}-N'-{(2'-desoxy-5'-O-dimethoxytrityl-β-D-ribofuranosyl)purin-6-yl}-O-[2-(p-nitrophenyl)ethyl]harnstoff

11 g N-{3'-O-[2-(p-Nitrophenyl)ethyl]-fluorescein-[2-(p-nitrophenyl)ethyl]ester-5-yl}-N'{(2'-desoxy-β-D-ribofuranosyl)purin-6-yl}-O-[2-(p-nitrophenyl)ethyl]harnstoff (als Rohprodukt 10 mmol) werden mit je 30 ml Pyridin 2 mal koevaporiert und in 100 ml abs. Pyridin gelöst. Nach Zugabe von 4,1 g Dimethoxytritylchlorid (12 mmol) wird 3 h bei RT gerürt, die Reaktionslösung bis auf ca. 50 ml eingeengt und mit 300 ml Methylenchlorid verdünnt. Nach Extraktion mit verd. Natriumhydrogencarbonatlösung wird über Magnesiumsulfat getrocknet, eingeengt, mit Toluol versetzt und erneut eingeengt und in 50 ml Methylenchlorid gelöst auf eine Kieselgelsäule aufgegeben. Die chromatographische Reinigung erfolgt mit 150 g Flash-SiO₂ in Toluol und einer Elutionsfolge von 200 ml Toluol/Essigester 8:2 ; 500 ml Toluol/Essigester 1:1 ; 195 ml Toluol/Essigester 1:1 + 5 ml Methanol ; 190 ml Toluol/Essigester 1:1 + 10 ml Methanol ; 185 ml Toluol/Essigester 1:1 + 15 ml Methanol ; 360 ml Toluol/Essigester 1:1 + 40 ml Methanol ; 450 ml Toluol/Essigester 1:1 + 50ml Methanol ; 300 ml Essigester/Methanol 8:2. Die Fraktionen 14-20 werden eingeengt, mit Ethanol versetz und erneut eingeengt und mit Ethanol im Ultraschallbad behandelt. Der resultierende Niederschlag wird über Nacht in Ethanol gerührt, danach abgesaugt und am Hochvakuum getrocknet. Gesamtausbeute: 12 g (8,7 mmol, 87%)
Smp.: Zersetzung ab 139°C
Rf= 0.42 (Kieselgel Tol/EE/MeOH 5:4:1)

### Beispiel 18

### N-{3'-O-[2-(p-Nitrophenyl)ethyl]-fluorescien-[2-(p-nitrophenyl)ethyl]ester-5-yl)-N'-{[(2'-desoxy-5'-O-dimethoxytrityl-β-D-ribofuranosyl)purin-6-yl]-3'-O-phosphorigsäure-2-(p-nitrophenylethyl)ester-N,N-diisopropylamid}-O-[2(p-nitrophenyl)ethyl]-harnstoff

500 mg N-{3'-O-[2-(p-Nitrophenyl)ethyl]-fluorescein-[2-(p-nitrophenyl)ethyl]ester-5-yl}-N'-{(2'-desoxy-5'-O-dimethoxytrityl-β-D-ribofuranosyl)purin-6-yl}-O-[2-(p-nitrophenyl)ethyl]-harnstoff (0,36 mmol) werden unter Schutzgasbedingungen in 7 ml abs. Methylenchlorid gelöst, mit 250 µl Ethyldiisopropylamin (1,4 mmol) und 242 mg (0,72 mmol) Phosphorigsäurechlorid-2-(p-nitrophenyl)ester-N,N-diisopropylamid versetzt und 1 h bei RT gerührt. Die resultierende braune Lösung wird mit Methylenchlorid/Natriumhydrogencarbonatlösung extrahiert, über Magnesiumsulfat getrocknet und eingeengt. Die restliche Hünig-Base wird am Hochvakuum abgezogen und das Rohprodukt chromatographisch gereinigt. Die Reinigung erfolgt mit 75 g neutralem Aluminiumoxid in Methylenchlorid und einer Elutionsfolge von 200 ml Methylenchlorid/Ethylacetat 1:1 ; 200 ml Ethylacetat ; 100 ml Ethylacetat + 3 ml Methanol ; 300 ml Ethylacetat/Methanol 95:5. Die Fraktionen 9-16 werden eingeengt und aus abs, Methylenchlorid aufgeschäumt. Ausbeute: 480 mg (0.29 mmol, 79%)
Rf= 0.3 (Kieselgel Tol/EE/MeOH 5:4,5:0,5)

### Beispiel 19

### N⁶-{3'-O-[2-(p-Nitrophenyl)ethyl]-fluorescein-2-(p-nitrophenyl)ethylester-5-yl-aminocarbonyl]-2'-desoxy-3',5'-tetrasopropyldisiloxan-1,3-diyl-adenosin

3 g N⁶-{Fluorescein-5-yl-aminocarbonyl}-2'-desoxy-3',5-tetraisopropyldisiloxan-1,3-diyl-adenosin (3.5 mmol), 3 g 2-(p-Nitrophenyl)ethanol (18 mmol) werden in 20 ml abs. Dioxan bei 60°C gerührt bis eine klare Lösung entstanden ist. Der tief roten Lösung werden 3 ml (18 mmol) Azodicarbonsäureethylester und 1.9 g Triphenylphosphin (7.3 mmol) zugesetzt und nach 10 min bei 60°C chromatographisch untersucht. Alles Edukt hat zu einem Hauptprodukt abreagiert, es sind nur Spuren Tris-Produkt entstanden. Zur Aufarbeitung wird das Reaktionsgemisch mit 300 ml Methylenchlorid verdünnt und mit 100 ml Phosphatpuffer pH7 extrariert. Die organische Phase wird abgetrennt, über Magnesiumsulfat getrocknet und zu einem orangefarbenen Öl eingeengt. Das Rohprodukt wird in 20 ml Toluol gelöst und auf eine Kieselgelsäule aufgetragen. Die chromatographische Reinigung erfolgt mit 70 g Flash-SiO₂ in Toluol und einer Elutionsfolge 200 ml Toluol ; 200 ml Toluol/Essigester 7/3 ; 200 ml Toluol/Essigester 1/1 ; 295 ml Toluol/Essigester 1/1 + 5 ml MeOH ; 380 ml Toluol/Essigester 1/1 + 20 ml MeOH ; 360 ml Toluol/Essigester 1/1 + 40 ml MeOH. Die Fraktionen 13-17 werden eingeengt, mit Ethanol versetzt und erneut eingeengt und mit Methanol im Ultraschallbad behandelt. Der resultierende Niederschlag wird noch ca. 1 h in Methanol gerührt, danach abgesaugt und am Hochvakuum getrocknet. Ausbeute: 2.6 g (2.2 mmol, 64%)
Rf= 0.78 (Kieselgel Tol/EE/MeOH 5:4:1)

### Beispiel 20

### N⁶-{3'-O-[2-(p-Nitrophenyl)ethyl]-fluorescein-2-(p-nitrophenyl)ethylester-5-yl-aminocarbonyl}-2'-desoxyadenosin

3 g N⁶-{3'-O-[2-(p-Nitrophenyl)ethyl]-fluorescein-2-(p-nitrophenyl)ethylester-5-yl-aminocarbonyl}-2'-desoxy-3',5'-tetraisopropyldisiloxan-1,3-diyl-adenosin (2.6 mmol) werden in 30 ml THF gelöst und mit 1,5 ml Essigsäure versetzt. Nach Zugabe von 2.1 g (6.6 mmol) TBAF (Trihydrat) wird die Mischung 16 h bei RT gerührt. Zur Aufarbeitung wird das Reaktionsgemisch mit 300 ml Methylenchlorid verdünnt und mit 100 ml NaHCO₃-Lösung extrahiert. Die organische Phase wird abgetrennt, über Magnesiumsulfat getrocknet und zu einem tief orangefarbenen Öl eingeengt. Das Rohprodukt wird mit Ethanol im Ultraschallbad behandelt, der resultierende Niederschlag abgesaugt. Dieses Produkt wird weiter umgesetzt. Zur Analyse werden 500 mg chromatographisch gereinigt. Elutionsfolge: 100 ml Methylenchlorid ; 200 ml Methylenchlorid/Methanol 98:2 ; 200 ml Methylenchlorid/ Methanol 95:5 ; 185 ml Methylenchlorid + 15 ml Methanol ; 185 ml Methylenchlorid + 15 ml Methanol ; 185 ml Methylenchlorid + 15 ml Methanol; 170 ml Methylenchlorid + 30 ml Methanol. Die Fraktionen 15-22 werden eingeengt, mit Ethanol versetzt und erneut eingeengt und mit Ethanol im Ultraschallbad behandelt. Der resultierende Niederschlag wird abgesaugt, mit Ether gewaschen und am Hochvakuum getrocknet. Ausbeute: 2.2g (2.4 mmol, 92%)
Rf= 0.25 (Kieselgel Tol/EE/MeOH 5:4:1)

### Beispiel 21

### N⁶-{3'-O-[2-(p-Nitrophenyl)ethyl]fluorescein-2-(p-nitrophenyl)ethylester-5-yl-aminocarbonyl)-2'-desoxy-5'-O-dimethoxytrityladenosin

1.5 g N⁶-{3'-O-[2-(p-Nitrophenyl)ethyl]-fluorescein-2-(p-nitrophenyl)ethylester-5-yl-aminocarbonyl}-2'-desoxyadenosin (2.6 mmol) werden mit je 10 ml Pyridin 2 mal eingeengt und in 20 ml abs. Pyridin gelöst Nach Zugabe von 610 mg Dimethoxytritylchlorid (1.8 mmol) wird 16 h bei RT gerührt, die Reaktionslösung mit 100 ml Methylenchlorid verdünnt. Nach Extraktion mit Natriumhydrogencarbonatlösung wird über Magnesiumsulfat getrocknet, eingeengt, mit Toluol versetzt und erneut eingeengt und in 10 ml Methylenchlorid gelöst auf eine Kieselgelsäule aufgegeben. Die chromatographische Reinigung erfolgt mit 50 g Flash-SiO₂ in Toluol und einer Elutionsfolge von 50 ml Toluol ; 200 ml Toluol/Essigester 1:1 ; 195 ml Toluol/Essigester 1:1 + 5 ml Methanol ; 190 ml Toluol/Essigester 1:1 + 10 ml Methanol ; 185 ml Toluol/Essigester 1:1 + 15 ml Methanol ; 185 ml Toluol/Essigester 1:1 + 15 ml Methanol ; 185 ml Toluol/Essigester 1:1 + 15 ml Methanol ; 150 ml Toluol/Essigester 1:1 + 50 ml Methanol. Die Fraktionen 10-12 werden eingeengt, mit Ethanol versetzt und erneut eingeengt und mit Ethanol im Ultraschallbad behandelt. Der resultierende Niederschlag wird über Nacht in Ethanol gerührt, danach abgesaugt und am Hochvakuum getrocknet. Die Fraktion 15 enthält noch Edukt. Ausbeuten: 1.5 g Produkt (1.2 mmol, 76%) ; 270 mg Edukt (0.3 mmol, 18%)
Rf= 0.57 (Kieselgel Tol/EE/MeOH 5:4:1)

### Beispiel 22

### N⁶-{3'-O-[2-(p-Nitrophenyl)ethyl]-fluorescein-2-(p-nitrophenyl)ethylester-5-yl-aminocarbonyl}-2'-desoxy-5'-O-dimethoxytrityladenosin-3'-O-phosphorigsäure-2-cyanoethylester-N,N-diisopropylamid

816 mg N⁶-{3'-O-[2-(p-Nitrophenyl)ethyl]-fluorescein-2-(p-nitrophenyl)ethylester-5-yl-aminocarbonyl}-2'-desoxy-5'-O-dimethoxytrityladenosin (0,66 mmol) und 12 mg Tetrazol (0,17 mmol) werden unter Schutzgasbedingungen in 2 ml abs. Methylenchlorid gelöst, mit 4 ml einer 0,25 M Stammlösung von Bis-[diisopropylamino]-2-cyanoethyloxyphosphan in abs. Methylenchlorid (1 mmol) versetzt und 24 h bei RT gerührt. Die Reaktionslösung wird mit 50 ml Chloroform verdünnt und zweimal mit je 50 ml Natriumhydrogencarbonatlösung extrahiert. Die org. Phase wird über Magnesiumsulfat getrocknet und bis auf ca. 5-10 ml eingeengt. Diese Lösung wird in 100 ml Ether/Petrolether (8:2) eingetropft. Der Niederschlag wird über eine Schutzgasfritte abgesaugt und am Hochvakuum getrocknet. Ausbeute: 800 mg (0,56 mmol, 85%)
Rf= 0.35 (Kieselgel Tol/EE/MeOH 5:4,5:0,5)

### Beispiel 23

### Herstellung eines spezifischen Oligonucleotids

Die Oligodesoxynucleotide werden an einem DNA-Synthezizer (Applied Biosystems 380B bzw. 392) unter Verwendung der Synthesezyklen des Herstellers bzw. publizierten Methoden (Tetrahedron Letters 31, 2549 (1990)) aufgebaut.
Die beschriebenen Phosphitamide werden in Methylenchlorid gelöst verwendet, die Kondensationszeit beträgt 300 sec. und es wird eine 0.3 M Lösung von Tetrazol in Acetonitril/Methylenchlorid (2:1) für die Kondensation mit den Farbstoff-modifizierten Phosphitamiden benötigt. Die Entfernung aller Schutzgruppen erfolgt am Träger durch eine 10 stündige Behandlung mit 1M DBU in Acetonitril und die Abspaltung vom Träger durch eine Behandlung mit 25% Ammoniak in Wasser. Die Rohprodukte werden lyophillisiert und bei Bedarf über HPLC gereinigt. Mit der beschriebenen Methode wird ein Oligonucleotid der Sequenz
5'- A* TCC CAG TCA CGA CGT-3' dargestellt.
Alle anderen Oligodesoxynucleotide können in entsprechender Weise dargestellt werden, wobei das markierte Nucleosid A* an einer beliebigen Stelle des Stranges eingebaut werden kann.

### Beispiel 24

### Hybridisierung mit markierter Sonde

Auf einer Nylonmembran werden unterschiedliche Mengen (0.1 pg-1 ng) U13 DNA aufgetragen, die zu der im Beispiel 23 hergestellten DNA-Sequenz komplementär ist. Nach Prehybridisierung des Filters für 1h bei 60°C in 6 X SSC (Natriumcitrat-Natriumchiorid-Puffer), 5 x Denhard (Rinderserumalbumin, Polyvinylpyrrolidon/Ficoll), 0,5 % SDS und 20 µg/ml denaturierte Heringssperma DNA erfolgt die Hybridisierung mit dem markieren Oligonucleotid aus Beispiel 23. Das Oligonucleotid wird in einer Konzentration von 1 mg/ml zugesetzt und bei 40°C 45 min. mit dem Filter hybridisiert. Anschließend wird das Filter nach bekannten Verfahren mit abnehmenden SSC-Konzentrationen gewaschen. Der Nachweis erfolgt über die Messung der Fluoreszenz auf dem Filter. Die gemessene Fluoreszenz ist der aufgetragenen DNA-Menge auf dem Filter proportional.

### Beispiel 25

### Hybridisierung und Detektion in Lösung

5 mg des gelabelten Oligonucleotids aus Beispiel 23 werden mit 1µg U13 DNA in 1 ml SSC auf Raumtemperatur abgekühlt. Die erfolgte Hybridisierung wird direkt in Lösung durch Messung der Fluoreszenzpolarisation detektiert.
Während eine Lösung mit dem Oligonucleotid alleine eine Polarisation von etwa 40 mp aufweist, steigt die Polarisation durch die Hybridisierung mit der U13 DNA auf 80 mp.

## Patentansprüche

1. Verbindungen der allgemeinen Formel I 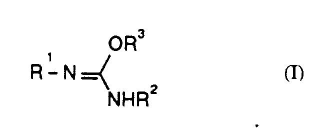 worin
R¹ = 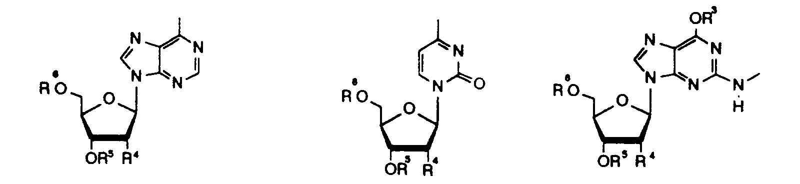 R² = ein Fluorophor
R³ = H, Alkyl mit 1-18 C-Atomen, -CH₂CH₂CN , 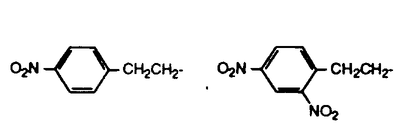 R⁴ = H, OH, OR⁵, OR⁶
R⁵, R⁶ = H, PO(OR⁷)(OR⁸), PS(OR⁷)(OR⁸), R⁸
R⁷ = H, Nucleosid, Nucleotid, Oligonucleotid
R⁸ = OH, übliche Schutzgruppen der Nucleotidchemie bedeuten.

2. Verbindungen der allgemeinen Formel I,
worin
R² = 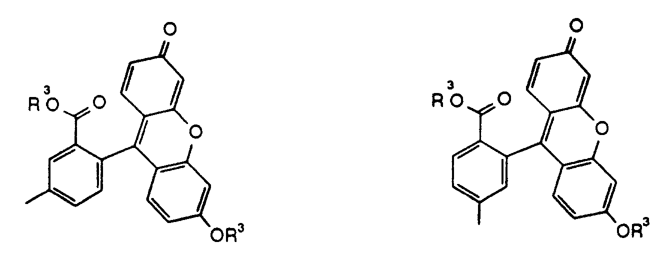 R³ = H, Alkyl mit 1-18 C-Atomen, -CH₂CH₂CN , 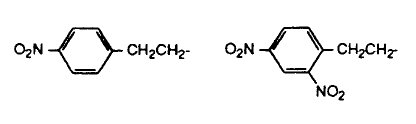 bedeuten.

3. Verfahren zur Herstellung der Verbindungen der allgemeinen Formel I, dadurch gekennzeichnet, daß man Verbindungen der allgemeinen Formel II
R¹-NH₂ (II)
worin R¹ die angegebene Bedeutung hat und R⁷ und R⁸ in der Nucleosidchemie übliche Schutzgruppen sind, mit Verbindungen der allgemeinen Formel III
R⁹-CO-R¹⁰ (III)
worin
R⁹ = Cl, Imidazol, Benzotriazol, 1.2.3.4-Tetrazol-, 1.2.3-Triazol-, 1.2.4-Triazolrest, die in 5-Stellung durch Arylreste substituiert sein können und
R¹⁰ = OR¹¹, Imidazol, Benzotriazol mit
R¹¹ = 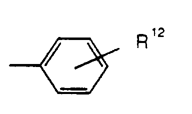 wobei R¹² = O-Alkyl, CN, NO₂, COO-Alkyl, Halogen bedeuten,
mit einem aromatischen Amin der allgemeinen Formel IV,
R²-NH₂ (IV)
worin R² die angegebene Bedeutung hat, umsetzt und die angegebenen Reste R³ einführt und anschließend die Schutzgruppen abgespaltet oder die Zuckerschutzgruppen direkt entfernt.

4. Verwendung der Verbindungen der allgemeinen Formel I zum Nachweis von DNA, RNA und deren Fragmenten.
